# EUROPÄISCHE PATENTANMELDUNG

(11) **EP 3 184 885 A1**
(43) Veröffentlichungstag der Anmeldung: **28.06.2017**
(21) Anmeldenummer: 15201956.8
(22) Anmeldetag: 22.12.2015
(51) Int. Cl.: F21V 8/00, G02B 6/26, G02B 5/02

(54) **FASEROPTISCHE STREUEINRICHTUNG UND HERSTELLUNGSVERFAHREN DAFÜR**

(71) Anmelder: Heraeus Quarzglas GmbH & Co. KG, 63450 Hanau (DE)
(72) Erfinder: SCHENK, Christian, 55218 Ingelheim (DE); SCHEICH, Gerrit, 63500 Seligenstadt (DE); TSCHOLITSCH, Nadine, 63457 Hanau (DE)
(74) Vertreter: Staudt, Armin Walter

(57) **Zusammenfassung**

Bei faseroptischen Streueinrichtungen wird über eine Lichtleitfaser aus Quarzglas Licht zu einem an einem distalen Endbereich der Lichtleitfaser vorgesehenen Diffusor übertragen. Der Diffusor enthält Streuzentren. Zur Erzeugung des Diffusors ist es bekannt, auf dem distalen Faserende der Lichtleitfaser eine Streumasse aufzubringen und diese zu dem Diffusor zu verfestigen. Um davon ausgehend ein Verfahren zur kostengünstigen und zuverlässigen Herstellung einer derartigen faseroptischen Streueinrichtung anzugeben, wird erfindungsgemäß vorgeschlagen, dass das Aufbringen der Streumasse folgende Verfahrensschritte umfasst
(a) Bereitstellen einer SiO₂-Körnung, die amorphe SiO₂-Teilchen enthält und die zu mindestens 90 Gew.-% aus SiO₂ besteht,
(b) Bereitstellen eines Hohlkörpers aus Glas mit einer Hohlraumwandung, die einen nach außen offenen Hohlraum umgibt,
(c) Bilden einer Schüttung der SiO₂-Körnung in dem Hohlraum und Einbringen des Faserendes in den Hohlraum, so dass mindestens ein Teil des Faserendes in die Schüttung hineinragt,
(d) thermisches Verdichten der Schüttung unter Ausbildung einer porenhaltigen und zu mindestens 90 Gew.-% aus SiO₂ bestehenden Sintermasse, die mindestens teilweise von einer Glashülle umgeben ist.

## Beschreibung

### Technisches Gebiet

Die vorliegende Erfindung betrifft eine faseroptische Streueinrichtung, mit einer Lichtleitfaser aus Quarzglas zur Übertragung von Licht zu einem an einem distalen Endbereich der Lichtleitfaser vorgesehenen Diffusor, der Streuzentren enthält.

Außerdem betrifft die Erfindung ein Verfahren zum Herstellen einer faseroptischen Streueinrichtung, bei der Licht in einer Lichtleitfaser aus Quarzglas geführt und über lichtstreuenden Diffusor abgestrahlt wird, wobei zur Erzeugung des Diffusors auf einem distalen Faserende der Lichtleitfaser eine Streumasse aufgebracht und diese zu dem Diffusor verfestigt wird.

Derartige faseroptische Streueinrichtungen sind mit einer oder mehreren Lichtleitfasern ausgestattet, die das von einer Lichtquelle emittierte Licht zu einem Bestrahlungs- oder Diagnoseort transportieren und dort in gewünschter Weise durch optische Streuung verteilen. Lichtleitfasern haben typischerweise einen Kern, der von einem Mantel umhüllt ist. Zum Schutz vor mechanischer Einwirkung können sie zusätzlich von einem äußeren Kunststoffmantel umhüllt sein.

### Stand der Technik

Bekannte Anwendungen umfassen die Bestrahlung von Oberflächen bei der Materialbearbeitung oder Restaurierung, die Beleuchtung von Proben in der Analytik und der Einsatz als Endoskope oder Fibroskope für in-vivo Beobachtungen und zum Durchführen medizinischer und therapeutischer Behandlungen.

Eine typische Anwendung in der Medizintechnik ist die sogenannte "photodynamischen Therapie" (PDT). Sie dient zur Bestrahlung einer Gewebeveränderung - beispielsweise einem Karzinom oder Tumor - mittels Licht geeigneter Wellenlänge. Dabei wird dem Patienten eine durch Lichtstrahlung aktivierbare Substanz (Photosensibilisator) verabreicht, die sich in der Gewebeveränderung anreichert. Die anschließende Bestrahlung löst photophysikalische Reaktionen aus, in der toxische Substanzen gebildet werden, die die Gewebeveränderung schädigen, während benachbarte Gewebebereiche weitgehend geschont werden. Die hierbei von der Faser zu übertragenden Lichtleistungen sind vergleichsweise niedrig, so dass optische Streueinrichtungen für dieses Gebiet mit Lichtleitfasern aus Kunststoff ausgestattet sein können.

Für die Behandlung oder Beleuchtung sphärischer Hohlräume, wie Blase oder chirurgisch geschaffene Hohlräume, wie sie nach Resektion der Masse eines Tumors verbleiben, sind faseroptische Streueinrichtungen mit sphärischem Streumuster vorteilhaft. Eine derartige Streueinrichtung und ein Verfahren für deren Herstellung sind in US 4,693,556 A beschrieben. Am distalen Faserende - also dem beim bestimmungsgemäßen Einsatz der Lichtquelle abgewandten Ende - wird der Faserkern freigelegt und poliert. Anschließend wird auf den Kernabschnitt und den angrenzenden Mantel solange schichtweise eine Streumasse aufgebracht, bis eine Streukugel gebildet ist. Die aus Quarzpartikeln und aus einem UV-aushärtbaren, optischen Adhäsiv bestehende Streumasse wird abschließend ausgehärtet.

Für die Behandlung oder Ausleuchtung länglicher Bereiche, wie beispielsweise der Bronchie oder der Speiseröhre, ist hingegen eine Lichtverteilung mit zylindrischer Geometrie besser geeignet. Diese wird beispielsweise mittels einer faseroptischen Streueinrichtung erzeugt, die ein zylindrisches Streumuster entlang eines Längenabschnitts des distalen Faserendes erzeugt, wie aus der US 5,074,632 A bekannt. Zur Herstellung der faseroptischen Vorrichtung wird der am distalen Ende einer optischen Faser (Lichtleitfaser) der Kern freigelegt, und anschließend werden der Kern und der daran angrenzenden Mantel mit einer streuenden Masse umhüllt. Die streuende Masse besteht aus einem UV-härtbaren Kleber, in den zwischen 5 bis 20 Gew.-% lichtstreuende Pulverteilchen eingebettet sind. Zur Minimierung von Reflexionsverlusten beim Austritt der Strahlung aus dem optischen Kern hat der Kleber einen Brechungsindex, der dem von Quarzglas entspricht (um 1,4). Die Pulverteilchen bestehen aus Al₂O₃, Diamant oder ZrO₂; sie sind für die Arbeitsstrahlung transparent und haben einen Brechungsindex, der höher ist als der von Quarzglas. Die streuende Masse wird mittels eine Pinsels aufgetragen und durch UV-Bestrahlung ausgehärtet. Zur mechanischen Stabilisierung wird die Streumasse mit einer durchsichtigen Schutzkappe ummantelt, die über ein Gewinde mit dem Kunststoff-Schutzmantel verbunden ist.

Ein weiteres typisches Anwendungsgebiet für faseroptische Streueinrichtungen ist die sogenannte "Laserablation". Dabei wird Material von einer Oberfläche durch Beschuss mit gepulster Laserstrahlung abgetragen. Die Laserstrahlung hat dabei eine hohe Leistungsdichte und bewirkt ein rasches Erhitzen und die Bildung eines Plasmas an der Bestrahlungsstelle. Die hohen Temperaturen und Energiedichten bei dieser Anwendung erfordern ein thermisch stabiles Fasermaterial, wie beispielsweise Quarzglas.

Die WO 2008/024397 A2 offenbart eine faseroptische Vorrichtung für die Laserablation mit einer Lichtleitfaser aus Quarzglas, die für hohe Lichtleistungen ausgelegt und an einem stirnseitigen Ende mit Streuzentren zum Verteilen des Lichts versehen ist. Die Streuzentren sind beispielsweise Nanohohlräume, die mit einem gepulsten Hochleistungslaser im Faserkern erzeugt werden. Bei einer anderen Ausführung wird ein Diffusor als separates Bauteil mit Kern-Mantelstruktur mittels Sol-Gel-Technik erzeugt und anschließend an das stirnseitige Ende der Lichtleitfaser angesetzt. Die Streuwirkung dieses Diffusors wird erreicht, indem in den Mantelbereich TiO₂-Streupartikel eingebaut werden. Das Diffusor-Bauteil ist stirnseitig verspiegelt und mit einer Schutzkappe versehen.

### Technische Aufgabenstellung

Bei faseroptischen Streueinrichtungen stellt sich die grundsätzliche Anforderung, dass die gestreute Lichtverteilung den Bearbeitungs-, Behandlungs- oder Beobachtungsort mit möglichst hoher Intensität, Zielgenauigkeit und Homogenität erreichen soll. Dafür sind Lichtleitfasern aus Glas besonders prädestiniert.

Andererseits müssen die mechanischen und optischen Eigenschaften sowohl während des bestimmungsgemäßen Einsatzes als auch während der sonstigen Handhabung, wie etwa Sterilisationsvorgängen zuverlässig gewährleistet sein. Es ist nicht akzeptabel, dass eine faseroptische Streueinrichtung während einer medizinischen Untersuchung oder Behandlung eines Patienten zerbricht. Insbesondere dieses Erfordernisses stellt für den Einsatz von Lichtleitfasern aus Glas in der Medizintechnik eine gewisse Hürde dar. Denn das Erzeugen oder Einbringen von Streuzentren im Bereich des Diffusors geht in aller Regel mit einer mechanischen Schwächung der ansonsten hochfesten Lichtleitfaser aus Glas einher und erhöht deren Bruchrisiko.

Der vorliegenden Erfindung liegt die Aufgabe zugrunde, eine faseroptische Streueinrichtung mit einer Lichtleitfaser aus Glas bereitzustellen, die besonders hohe Anforderungen an die mechanische Belastbarkeit erfüllt.

Weiterhin liegt der Erfindung die Aufgabe zugrunde, ein Verfahren zur kostengünstigen und zuverlässigen Herstellung einer derartigen faseroptischen Streueinrichtung anzugeben.

### Allgemeine Beschreibung der Erfindung

### Faseroptische Streueinrichtung

Hinsichtlich der faseroptischen Streueinrichtung wird diese Aufgabe ausgehend von einer Einrichtung der eingangs genannten Gattung erfindungsgemäß dadurch gelöst, dass mindestens ein Teil der Streuzentren als geschlossene Poren in einer zu mindestens 90 Gew.-% aus SiO₂ bestehenden Sintermasse ausgebildet sind, die mindestens teilweise von einer Glashülle umgeben ist.

Die Glashülle und die darin eingeschlossene Sintermasse bilden den Diffusor oder einen Teil desselben. Im Unterschied zum Stand der Technik ist der Diffusor bei der faseroptischen Streueinrichtung gemäß der Erfindung somit ein Verbundkörper aus einer im Wesentlichen aus SiO₂ bestehenden Sintermasse, die Streuzentren in Form von Poren enthält und einer Glashülle, die die SiO₂-Sintermasse mindestens teilweise umgibt.
(a) Die in der Glashülle eingeschlossene Sintermasse ist Ergebnis eines Sinterprozesses, bei dem eine aus SiO₂-haltigen Partikeln gebildete Schüttung innerhalb der Glashülle geformt und so erwärmt wird, dass sich die SiO₂-haltigen Partikel miteinander verbinden ohne vollständig aufzuschmelzen. Die enthält offene Poren. Bei offener Porosität ist das Porenvolumen in der Sintermasse so groß, dass die meisten Poren miteinander verbunden sind. Sie bilden mindestens einen Teil der Streuzentren des Diffusors. Die Streuwirkung der Sintermasse beruht im Wesentlichen auf den offenen Poren, und nicht etwa auf einem Streumittel aus einem anderen Werkstoff oder auf Streuung an Partikel-Oberflächen, die in eine Kunststoff-Matrix eingebettet sind. Dadurch ist es möglich, eine Sintermasse mit hohem SiO₂-Anteil bereitzustellen, der mindestens 90 Gew.-%, vorzugsweise mindestens 95 Gew.-% beträgt.
(b) Das große Porenvolumens und die offene Porosität tragen dazu bei, dass die Sintermasse auch auf kurzer Lichtübertragungsstrecke eine ausgeprägte Streuwirkung erzeugt. Ein Porenvolumen von mehr als 30% führt zu einer geringen mechanischen Stabilität und ist daher nicht bevorzugt.
(c) Andererseits sind nach außen offene Poren sind für viele Anwendungen der faseroptischen Streueinrichtung nicht akzeptabel. Daher ist die Sintermasse von außen mittels einer Glashülle abgeschlossen, eine dichte, porenfreie Oberfläche hat. Sie schirmt die Sintermasse vor der Umgebung ab. Dadurch bewahrt sie zum einen die Sintermasse vor Beschädigung, Korrosion oder Ablagerungen, und zum anderen vermindert die Glashülle die Gefahr einer von der Sintermasse ausgehenden Beschädigung oder Kontamination des Einsatzortes. Dadurch können kann das Streuungsverhalten der Sintermasse optimiert werden, und die Anforderungen an die mechanische Festigkeit und an die Inertheit der Sintermasse können niedriger sein, als dies ohne die Glashülle der Fall wäre. Insbesondere kann die Porosität der Sintermasse hoch sein und beispielsweise mehr als 10 % betragen, so dass sich infolge der vielfachen Poren eine besonders hohe Streuwirkung einstellt. Zur Erzielung dieser Wirkungen genügen geringe Wandungsstärken, so dass die Glashülle vorzugsweise eine maximale Dicke von weniger als 1 mm aufweist.
(d) Die Lichtleitfaser umfasst als obligatorische Lichtleitfaser-Komponente mindestens einen Kern und als fakultative Lichtleitfaser-Komponente mindestens einen den Kern umhüllenden Mantel. Der Kern und gegebenenfalls der Mantel bestehen aus dotiertem oder aus undotiertem hochkieselsäurehaltigem Glas, das hier und im Folgenden auch als Quarzglas bezeichnet wird. Der SiO₂-Anteil des Quarzglases beträgt somit mindestens 90 Gew.-%. Die Sintermasse hat mindestens eine gemeinsame Kontaktfläche mit einer oder beiden Lichtleitfaser-Komponenten. Die die Kontaktfläche bildenden Werkstoffe haben somit eine ähnliche, im Idealfall die gleiche chemische Zusammensetzung; sie bestehen jeweils aus dotiertem oder undotiertem Quarzglas, wobei sie sich in ihrem SiO₂-Gewichtsanteil um maximal 10 Prozentpunkte, vorzugsweise um maximal 5 Prozentpunkte voneinander unterscheiden. Die an die gemeinsame Kontaktfläche grenzenden Werkstoffe (Lichtleitfaser-Komponente / Sintermasse) sind in ihrer chemischen Zusammensetzung somit ähnlich, was im Folgenden auch als "arteigen" bezeichnet wird.
   Die Arteigenheit bewirkt, dass auch der thermische Ausdehnungskoeffizient und dessen Temperaturabhängigkeit beiderseits ähnlich und im Idealfall gleich sind. Dies vermindert das Auftreten mechanischer Spannungen und temperaturbedingter Verformungen beim bestimmungsgemäßen Einsatz der Streueinrichtung, was sich auch in einer hohen Temperaturwechselbeständigkeit und gegebenenfalls einer guten Haftung einer Sintermasse auf der Lichtleitfaser-Komponente zeigt. Die faseroptische Streueinrichtung zeichnet sich daher durch hohe mechanische und optische Stabilität im Einsatz aus.
(e) Dazu trägt auch die geometrische Form der Poren bei. Die Poren entstehen beim Sintern einer Körnungs-Schüttung zu der Sintermasse oder sie bleiben nach dem Sintern als Reste vorhandener, größerer Hohlräume erhalten. Die Anzahl und Größe der Poren in der Sintermasse hängt von der Sintertemperatur und -dauer ab. Durch gasbildende Komponenten in der Schüttung kann der Porositätsgrad erhöht werden. Die gasbildenden Komponenten, wie etwa Kohlenstoff, Siliciumcarbid oder Siliciumnitrid, zersetzen sich beim Sintern oder sie reagieren mit Sauerstoff unter Bildung flüchtiger Substanzen und verschwinden vorzugsweise ohne nennenswerten Rückstand. Herstellungsbedingt - das heißt, infolge ihrer Ausformung beim Sintern der Schüttung - zeigen die Poren ein kleines Aspektverhältnis von weniger als 3 und die Wandungen der Poren sind in aller Regel abgerundet. Bei mechanischer Belastung auftretende Spannungsspitzen sind daher geringer als bei gleich großen aber scharfkantigen Hohlräumen und Streupartikeln.

Die "Porosität" oder das "Porenvolumen" eines porösen Materials bezeichnet das von Hohlräumen belegte, freie Volumen innerhalb des Materials. "Offene Porosität" eines Materials zeigt sich darin, dass es saugfähig ist, was anhand eines Farbeindringtests nachweisbar ist. Diese Nachweismethode erfordert gegebenenfalls das vorherige Beseitigen der dichten Glashülle.

Die Sintermasse hat mehr oder wenige sphärische Poren mit einem kleinen "Aspektverhältnis" (auch als "Strukturverhältnis" bezeichnet). Darunter wird hier das Verhältnis von größter und kleinster Porenabmessung verstanden. Ein Aspektverhältnis von maximal 3 bedeutet demnach, dass die größte Porenabmessung nicht mehr als 3-mal größer ist als seine kleinste Porenabmessung. Sphärische Poren zeigen im Vergleich zu langgestreckten Hohlkanälen eine räumliche Streuwirkung.

Die Sintermasse bildet eine Schicht am distalen Ende der Lichtleitfaser. Sie hat mindestens eine Kontaktfläche mit der Stirnseite der Lichtleitfaser und/oder mit deren Zylindermantel.

Im Hinblick auf hohe mechanische Stabilität und eine hohe Effizienz der ausgekoppelten Streustrahlung ist die Sintermasse in Kontakt mit einem Faserkern, wobei sich die Zusammensetzungen von Sintermasse und Faserkern in ihrem SiO₂-Gehalt um 1 Prozentpunkt oder weniger, vorzugsweise um weniger als 0,5 Prozentpunkte, unterscheiden

Eine bevorzugte Ausführungsform der faseroptischen Streueinrichtung ist dadurch gekennzeichnet, dass die Glashülle aus Quarzglas besteht und mit der Sintermasse integral verbunden ist.

Die Glashülle liegt mindestens bereichsweise unmittelbar auf der Sintermasse auf und ist mit dieser integral verbunden, sie ist beispielsweise mit der Sintermasse verschmolzen. Der integrale Verbund trägt zu einer hohen mechanischen Stabilität des Diffusors bei und verbessert die Reproduzierbarkeit der Streueigenschaften.

Die Streuwirkung des Diffusors steigt mit der Porosität der Sintermasse. Die mit der Sintermasse verbundene Glashülle ist dicht und mindestens an ihrer Außenseite blasenfrei. Sie ermöglicht eine Ausgestaltung der Sintermasse mit besonders hoher Porosität von 10 % oder mehr, vorzugsweise mindestens 15%. Infolge des hohen Porenvolumens und der Vielzahl der Poren stellt sich eine besonders hohe Streuwirkung ein.

Bei einer bevorzugten Ausführungsform der faseroptischen Streueinrichtung ist das distale Ende der Lichtleitfaser nicht zylinderförmig, sondern es ist kegelförmig ausgebildet. Diese Gestaltung des Faserendes beeinflusst die Auskopplung des im Faserkern geführten Lichtes. Das Licht wird früher und über eine längere Strecke aus dem Faserkern ausgekoppelt, als dies ohne diese Gestaltung der Fall wäre. Durch diese Gestaltung kann die Streuintensität über eine längere Strecke größere Länge homogenisiert werden.

### Verfahren zur Herstellung einer faseroptischen Streueinrichtung

Hinsichtlich des Verfahrens zur Herstellung einer faseroptischen Streueinrichtung wird die oben angegebene Aufgabe ausgehend von einem Verfahren der eingangs genannten Gattung erfindungsgemäß dadurch gelöst, dass das Aufbringen der Streumasse folgende Verfahrensschritte umfasst
(a) Bereitstellen einer SiO₂-Körnung, die amorphe SiO₂-Teilchen enthält und die zu mindestens 90 Gew.-% aus SiO₂ besteht,
(b) Bereitstellen eines Hohlkörpers aus Glas mit einer Hohlraumwandung, die einen nach außen offenen Hohlraum umgibt,
(c) Bilden einer Schüttung der SiO₂-Körnung in dem Hohlraum und Einbringen des Faserendes in den Hohlraum, so dass mindestens ein Teil des Faserendes in die Schüttung hineinragt,
(d) thermisches Verdichten der Schüttung unter Ausbildung einer porenhaltigen und zu mindestens 90 Gew.-% aus SiO₂ bestehenden Sintermasse, die mindestens teilweise von einer Glashülle umgeben ist.

Das Verfahren dient dazu, am distalen Endbereich der Lichtleitfaser einen Diffusor aufzubringen, der als Verbundkörper aus einer Glashülle und einer porösen Sintermasse vorliegt, wobei die Glashülle die poröse Sintermasse mindestens teilweise, vorzugsweise vollständig umschließt.

Zum Aufbringen des Diffusors werden in einen Hohlraum, der von einem Hohlkörper aus Glas, bevorzugt aus Quarzglas, bereitgestellt wird, das Ende der Lichtleitfaser sowie eine Schüttung rieselfähiger SiO₂-Körnung eingebracht, die amorphe SiO₂-Teilchen enthält und die zu mindestens 90 Gew.-% aus SiO₂ besteht. Ein Ende der Lichtleitfaser taucht dabei so in die Schüttung ein, dass es mindestens stirnseitig, einseitig oder allseitig von der amorphen SiO₂-Körnung umgeben ist. Bevorzugt wird zunächst die Schüttung in den Hohlraum eingebracht und danach das Lichtleitfaser-Ende in die Schüttung eingeführt.

Die Schüttung der amorphen SiO₂-Körnung wird anschließend innerhalb des Hohlraums derart erhitzt, dass sich eine offen-poröses Sintermasse ausbildet. Dabei kann sich der Hohlkörper verformen und auf die Sintermasse aufschmelzen. Es bildet sich ein Diffusor aus Sintermasse und Glashülle. Der Hohlkörper ist beispielsweise ein einseitig verschlossenes Rohr (eine Kapillare) oder eine Hohlkugel.

Die Sintermasse ist somit Ergebnis einer thermischen Verdichtung, bei der die Schüttung aus SiO₂-haltigen Teilchen so erwärmt wird, dass sich die SiO₂-haltigen Teilchen miteinander verbinden ohne vollständig aufzuschmelzen, was hier auch als "Sintern" bezeichnet wird. Die ursprünglichen SiO₂-Teilchen sind über sogenannte "Sinterhälse" miteinander verbunden und bilden ein poröses, zusammenhängendes Gerüst. Sintertemperatur und -dauer sind so zu wählen, dass eine Sintermasse mit offener Porosität erhalten wird. Die Poren bilden mindestens einen Teil der Streuzentren des Diffusors. Diese besteht aus bevorzugt nur aus Quarzglas und aus Poren; Partikel zur Erzeugung der Streuwirkung sind nicht erforderlich. Dadurch kann der Werkstoff der Sintermasse einen hohen SiO₂-Anteil von mindestens 90 Gew.-%, vorzugsweise mindestens 95 Gew.-% aufweisen. Die Kontaktfläche zwischen der Sintermasse und dem der Quarzglas-Lichtleitfaser wird somit von Werkstoffen gebildet, die eine ähnliche, im Idealfall die gleiche chemische Zusammensetzung haben. Sie bestehen jeweils aus dotiertem oder undotiertem Quarzglas, wobei sie sich in ihrem SiO₂-Gewichts-anteil um maximal 10 Prozentpunkte, vorzugsweise um maximal 5 Prozentpunkte voneinander unterscheiden. Diese "Arteigenheit" bewirkt, dass auch der thermische Ausdehnungskoeffizient und dessen Temperaturabhängigkeit beiderseits ähnlich und im Idealfall gleich sind. Dies vermindert das Auftreten mechanischer Spannungen und temperaturbedingter Verformungen beim bestimmungsgemäßen Einsatz der Streueinrichtung, was sich auch in einer hohen Temperaturwechselbeständigkeit und einer guten Haftung der Sintermasse auf der Lichtleitfaser zeigt. Die faseroptische Streueinrichtung zeichnet sich daher durch hohe mechanische und optische Stabilität im Einsatz aus.

Die Porenbildung beim Sintern der Schüttung der SiO₂-Körnung hängt von der Sintertemperatur und -dauer ab. Durch einen Zusatz gasbildender Komponenten zu der Schüttung kann der Porositätsgrad erhöht werden. Die gasbildenden Komponenten, wie etwa Kohlenstoff oder Siliciumnitrid, zersetzen sich beim Sintern oder sie reagieren mit Sauerstoff unter Bildung flüchtiger Substanzen und verschwinden vorzugsweise ohne nennenswerten Rückstand.

Herstellungsbedingt sind die Wandungen der Poren in aller Regel abgerundet. Bei mechanischer Belastung auftretende Spannungsspitzen sind daher geringer als bei gleich großen aber scharfkantigen Hohlräumen und bei Einsatz von Streupartikeln. Auch diese Maßnahme trägt zu der hohen mechanischen Stabilität der Streueinrichtung bei.

Die amorphe SiO₂-Körnung setzt sich beispielsweise aus SiO₂-Ruß (häufig auch als "SiO₂-Soot" bezeichnet), aus Granulaten aus derartigem SiO₂-Soot, beispielsweise Sprühgranulat oder plasmageschmolzenes Granulat, aus sphärischer dichter SiO₂-Körnung, gebrochene Körnung oder aus Mischungen dieser Komponenten zusammen.

Sphärische Körnung zeigt eine vergleichsweise hohe Rieselfähigkeit. Bei gebrochener Körnung zeigen die SiO₂-Teilchen eine splittrige Morphologie. Sie werden erhalten, indem gröbere Quarzglas-Körner oder Quarzglas-Bruchstücke nass oder trocken vermahlen werden. Im Vergleich zu einer sphärischen Morphologie zeigen splittrige SiO₂-Teilchen eine bessere Verzahnung innerhalb der Schüttung, was die Schüttdichte der Schüttung verringert und die Sinteraktivität erhöht. Es hat sich auch gezeigt, dass gebrochene SiO₂-Körnung einen höheren Beitrag zur Streuung des Lichtes liefert. Durch Sieben der Körnung wird eine enge Korngrößenverteilung erzeugt und dadurch die Schüttdichte (der Füllgrad) der Schüttung im Vergleich zum Einsatz einer breiten oder mehrmodalen Korngrößenverteilung verringert.

Granulatteilchen liegen als Agglomerat einzelner sphärischer SiO₂-Primärteilchen vor. Die mittlere Teilchengröße der SiO₂-Primärteilchen liegt bei etwa 50 nm und sie werden hier auch als SiO₂-Nanoteilchen bezeichnet. Das Agglomerat der SiO₂-Primärteilchen ist lose, so dass es durch leichten mechanischen Druck zerrieben und zerkleinert werden kann. Zwischen den SiO₂-Primärteilchen sind offene Porenkanäle ausgebildet. Die spezifische Oberfläche (nach BET) von Sprühgranulat liegt typischerweise um 30 m²/g und die Schüttdichte liegt bei 0,8 kg/l.

Das Einbringen der amorphen SiO₂-Körnung in den Hohlraum des Hohlkörpers wird erleichtert, wenn die SiO₂-Körnung eine hohe Rieselfähigkeit aufweist. Die Rieselfähigkeit ist ein Maß für die Beweglichkeit oder das Fließverhalten der Körnung. Sie wird von Korngröße und Korngrößenverteilung beeinflusst. Die Messung erfolgt nach DIN EN ISO 6186 durch Ermittlung der Auslaufdauer der Körnung aus genormten Prüftrichtern mit verschiedenen Auslauföffnungsgrößen. Die spezifische Auslaufdauer ergibt sich als Mittelwert dreier Einzelmessungen.

Ein weiteres Problem ergibt sich dadurch, dass die Sintermasse aus SiO₂-Teilchen erzeugt wird, das einerseits für die thermische Verfestigung einer vergleichsweise hohen Sintertemperatur bedarf, dass aber andererseits die Lichtleitfaser, auf der die Sintermasse erzeugt wird möglichst wenig thermisch belastet werden soll. Das Sintern wird noch durch die Abschirmung der zu sinternden Körnung durch den Hohlkörper aus Glas erschwert, der als Wärmeisolator wirkt.

Im Hinblick darauf hat sich eine amorphe SiO₂-Körnung bewährt, die SiO₂-Teilchen mit Teilchengrößen im Bereich bis maximal 500 µm, vorzugsweise maximal 350 µm enthält, wobei SiO₂-Teilchen mit Teilchengrößen im Bereich zwischen 20 µm und 300 µm - bevorzugt SiO₂-Teilchen mit Teilchengrößen im Bereich zwischen 100 µm und 200 µm - den größten Volumenanteil ausmachen.

Große SiO₂-Teilchen zeigen tendenziell scharfe Reflexe im gestreuten Licht; kleine SiO₂-Teilchen verbessern die Diffusorwirkung, vermindern aber auch die von Diffusor emittierte Intensität des Streulichtes. Der genannte Größenbereich und die Teilchengrößenverteilung stellen einen geeigneten Kompromiss zwischen scharfen Reflexen des gestreuten Lichtes und Diffusorwirkung dar. Außerdem ergeben derartige Schüttungen nicht nur eine akzeptable Rieselfähigkeit, sondern auch ein vorteilhaftes Sinterverhalten und eine vergleichsweise geringe Schwindung beim Sintern.

Insbesondere das Sinterverhalten wird noch dadurch verbessert, dass die amorphe SiO₂-Körnung SiO₂-Nanoteilchen in einem Gewichtsanteil - bezogen auf den gesamten Feststoffgehalt - im Bereich von 1 bis 10 % enthält.

Die SiO₂-Nanoteilchen können zum Beispiel durch Pyrolyse oder Hydrolyse siliziumhaltiger Ausgangsverbindungen oder durch Polykondensation polymerisierbarer Siliziumverbindungen hergestellt werden. Nanoteilchen bestehen typischer Weise aus einem Verbund einiger Tausend SiO₂-Moleküle und haben üblicherweise eine spezifische Oberfläche nach BET im Bereich von 50 bis 400 m²/g. Die amorphe SiO₂-Körnung weist gegebenenfalls eine mehrmodale Teilchengrößenverteilung auf, mit einem ersten Maximum der Größenverteilung (D₅₀-Wert) im Bereich von weniger als 100 nm, der auf den Anteil an SiO₂-Nanoteilchen zurückzuführen ist, und mit einem zweiten Maximum der Größenverteilung (D₅₀-Wert) im Bereich oberhalb von 1 µm. Diese Teilchengrößenverteilung erleichtert die Einstellung einer hohen Feststoffdichte der Schüttung, wodurch die Schrumpfung beim Sintern und damit die Gefahr einer Rissbildung weiter vermindert werden. Durch den Zusatz der SiO₂-Nanoteilchen werden die Oberfläche des Feststoffs und die Sinteraktivität erhöht. Bei einem Anteil von weniger als 1 Gew.-% dieser Teilchen im Schlicker wirken sich die Nanoteilchen auf die Vergrößerung der Feststoffoberfläche nicht nennenswert aus, wohingegen Gehalte von mehr als 10 Gew.-% zu einer verstärkten Schrumpfung beim Sintern führen, die ein defektfreies Sintern erschweren kann.

Erfindungsgemäß wird eine Sintermasse mit hoher, offener Porosität angestrebt. Dies wird vorzugsweise dadurch erreicht, dass eine Schüttung mit einem Füllgrad von weniger als 85 %, bevorzugt im Bereich von 60 bis 80 %, erzeugt wird.

Der Füllgrad eines porösen Materials bezeichnet das von Feststoff belegte Volumen innerhalb eines porösen Materials. Er entspricht dem inversen Porenvolumen und ist ein Maß für die Schüttdichte. Die aus der Schüttung erzeugte Sintermasse hat in jedem Fall eine hohe Porosität von mindestens 15 %. Bei einer Schüttung mit einem Füllgrad von weniger als 60 % erweist sich jedoch das thermische Verdichten durch Sintern als schwierig. Bei einem Füllgrad von mehr als 85 % ergibt sich ein vergleichsweise geringes Porenvolumen in der Sintermasse.

Es hat sich als vorteilhaft erwiesen, wenn eine Schüttung erzeugt wird, die zwischen dem Faserende und der Hohlraumwandung eine Schüttungsschicht mit einer Schichtdicke im Bereich von 0,1 bis 3 mm aufweist.

Je größer die Schichtdicke ist, umso größer ist bei gleicher Teilchengröße die Streuwirkung. Die genannte Untergrenze von 0,1 mm ergibt sich aus der Erkenntnis, dass sie mindestens dem Fünffachen, vorzugsweise mindestens dem Zehnfachen der mittleren Teilchengröße (D₅₀-Wert) entsprechen sollte. Mit der entsprechenden Anzahl von Wechselwirkungen des aus dem Faserkern austretenden Lichts wird eine Diffusorwirkung ohne scharfe Reflexe erhalten. Mit zunehmender Dicke nimmt die Intensität des vom Diffusor emittierten Lichtes ab, was zur bevorzugten Obergrenze von 3 mm führt.

Bei einer vorteilhaften Verfahrensvariante wird innerhalb der Schüttungsschicht ein axialer Gradient in der Dichte der Schüttung oder in der Zusammensetzung der SiO₂-Körnung eingestellt, derart, dass sich nach dem thermischen Verdichten der Schüttung ein Dichtegradient zwischen einer geringeren Dichte in einem faserendnahen Bereich und einer höheren Dichte in einem faserendfernen Bereich des Hohlkörpers einstellt.

Der Dichtegradient der Schüttung führt zu einem Dichtegradienten in der Sintermasse. Ebenso bewirkt ein Gradient in der chemischen Zusammensetzung der SiO₂-Körnung einen entsprechenden Gradienten in der Sintermasse. Mittels eines derartigen Gradienten können Streuzentren inhomogen über die Dicke der Sintermasse verteilt und die Streuwirkung an die Eindringtiefe des einfallenden Lichtes angepasst werden. So vermindert eine in axialer Richtung zunehmende Dichte den Strahlungstransport in dieser Richtung und begünstigt stattdessen eine seitliche Abstrahlung. Zur Einstellung des Dichtegradienten kann beispielsweise die Körnung am faserendfernen Bereich des Hohlkörpers einen höheren Feinanteil haben als am faserendnahen Bereich. Unter Nutzung des sogenannten "Paranuss-Effekts" kann der Feinanteil im unteren Bereich der Schüttung durch Vibration angereichert werden.

Weiterhin hat sich eine Verfahrensvariante bewährt, bei der die SiO₂-Körnung eine erste Körnungsart mit einem ersten Brechungsindex und einer ersten Viskosität sowie zweite Körnungsart mit einem zweiten Brechungsindex und einer zweiten Viskosität aufweist, wobei sich der erste Brechungsindex und der zweite Brechungsindex voneinander unterscheiden und wobei die zweite Viskosität niedriger ist als die erste Viskosität.

Die erste und die zweite Körnungsart bestehen beispielsweise aus Quarzglas mit unterschiedlichen Dotierstoffen und/oder mit verschiedenen Konzentrationen desselben Dotierstoffs. In der Mischung bewirken die Unterschiede im Brechungsindex eine erhöhte Streuwirkung. Dadurch, dass eine der Körnungsarten eine geringere Viskosität aufweist als die andere, wirkt sie quasi als Sinterhilfsmittel für das Sintern auch der anderen Körnungsart. Dies trägt zu einer Verringerung der Sintertemperatur bei. Als Dotierstoff werden vorzugsweise Glasbildner eingesetzt, wie etwa B₂O₃, GeO₂ oder P₂O₅. Die zweite Körnungsart ist vorzugsweise gemahlenes Glaspulver beispielsweise aus Borosilikat-, Boroalumosilikat-, Alumosilikat- oder Bismutsilikatglas. Die zweite Körnungsart hat eine niedrigere Viskosität und schmilzt dadurch beim Sintern früher als die erste Körnungsart; und sie wirkt dabei als "Binder" zwischen den Körnern der ersten Körnungsart, was die Sintertemperatur merklich senkt. Dabei hat es sich bewährt, wenn der Volumenanteil der zweiten Körnungsart weniger als 5% am Gesamt-Volumen der SiO₂-Körnung beträgt.

Das thermische Verdichten der Schüttung erfolgt lokalisiert am Ende der Lichtleitfaser durch Erhitzen mittels einer Brennerflamme oder in einem Ofen. Um eine Beeinträchtigung der Lichtleitfaser beim Sintern möglichst zu vermeiden, erfolgt das thermische Verdichten der Schüttung jedoch vorzugsweise durch Erhitzen in einem Ofen, mittels eines Plasmas, eines Lasers oder durch eine Brennerflamme (Heißgas).

Mittels einer Mikroplasma-Einrichtung kann ein in seinem Energiegehalt in weiten Bereichen einstellbarer Plasmastrahl auf einen kleinen Raum einwirken. Durch Fokussierung eines Lasers kann das Erhitzen lokal auf de zu sinternde Schüttung beschränkt werden, so dass die übrige Mikrostruktur der faseroptischen Streueinrichtung im Wesentlichen unbeeinflusst bleibt. Das Erhitzen mittels Heißgas ist einfacher und schneller und hat sich insbesondere bewährt, wenn das die SiO₂-Körnung viskositätsverringernde Dotierstoffe enthält.

Im Hinblick auf die Aufrechterhaltung offener Porosität der Sintermasse hat sich bewährt, wenn das Sintern ein Erhitzen bei einer Temperatur im Bereich von 1000 bis 1500 °C umfasst. Durch Zusätze, die beim Erhitzen Gase freisetzen können, wie etwa SiC, Si₃N₄ oder Kohlenstoff, kann die Porosität der Sintermasse vergrößert, auf einen hohen Wert eingestellt und einem versehentliche Dichtsintern entgegengewirkt werden. Nach dem thermischen Verdichten kann eine mechanische Nachbearbeitung der Oberfläche der Sintermasse erfolgen, etwa um die geometrische Gestalt zu optimieren oder um die Oberfläche der Sintermasse zu glätten oder zu strukturieren.

### Definitionen

Einzelne Produktmerkmale und Verfahrensschritte und Begriffe der obigen Beschreibung werden im Folgenden ergänzend definiert. Die Definitionen sind Bestandteil der Beschreibung der Erfindung. Bei einem sachlichen Widerspruch zwischen einer der folgenden Definitionen und der übrigen Beschreibung ist das in der Beschreibung Gesagte maßgeblich.

"Synthetisches Quarzglas" besteht aus undotiertem oder dotiertem SiO₂, das vorzugsweise durch Hydrolyse oder Oxidation eines siliziumhaltigen Ausgangsmaterials erzeugt worden ist. Als Ausgangsmaterialien kommen halogenierte Siliziumverbindungen, wie etwa SiCl₄, SiHCl₃ und Alkoxyde (Tetramethoxydsilan, TMOS, Methyltrimethoxysilan, MTMS) oder Siloxane (wie etwa Polyalkylsiloxane, beispielswiese Octame-thylzyklotetrasiloxan OMCTS) oder Mischungen der genannten Ausgangsmaterialien in Frage.

Durch Hydrolyse oder Oxidation werden "SiO₂-Primärteilchen" im nanoskaligen Bereich mit einem SiO₂-Gehalt von mehr als 99,99 % erhalten. Diese Teilchen werden auch als SiO₂-Nanoteilchen bezeichnet. In ihrer Gesamtheit bilden diese ein feinteiliges "SiO₂-Pulver", das auch als "Sootstaub", "Soot" oder "Ruß" bezeichnet wird. Dessen spezifische Oberfläche (BET) liegt im Bereich von 25 bis 55 m²/g.

SiO₂-Pulver ist hochrein in dem Sinne, dass die Hauptkomponente SiO₂ ist und nur geringe Mengen an anderen Elementen im ppm- oder ppb-Bereich enthalten sind. Verunreinigungen in dem SiO₂-Pulver oder in dem daraus hergestellten Quarzglas werden mittels ICP-OES oder ICP-MS-Methoden ermittelt, wobei die Konzentration in Gewichts-Anteilen angegeben wird.

Die "spezifische Oberfläche (BET)" wird nach der Methode von Brunauer, Emmet und Teller (BET) anhand der DIN 66132 ermittelt und basiert auf Gasabsorption an der zu messenden Oberfläche.

Beim Granulieren bilden sich durch Zusammenlagerungen von Teilchen des SiO₂-Pulvers diskrete, größere Agglomerate, die als "SiO₂-Granulatteilchen" oder kurz "Granulatteilchen" bezeichnet werden. In ihrer Gesamtheit bilden die Granulatteilchen ein "SiO₂-Granulat".

Man kann zwischen Aufbaugranulation und Pressgranulation und verfahrenstechnisch zwischen Nass- und Trocken-Granulierverfahren unterscheiden. Bekannte Methoden sind Rollgranulation in einem Granulierteller, Sprühgranulation, Zentrifugalzerstäubung, Wirbelschichtgranulation, Granulierverfahren unter Einsatz einer Granuliermühle, Kompaktierung, Walzenpressen, Brikettierung, Schülpenherstellung oder Extrudierung.

Die "Partikelgröße der Granulatteilchen" bezeichnet die makroskopische Abmessung eines jeden festen Granulat-Teilchens und wird als Partikelgrößenverteilung mittels Siebmethoden ermittelt. Bei der Siebanalyse wird das Pulver mittels Siebböden mit unterschiedlichen Siebgrößen separiert. Der Gewichtsanteil des Siebgutes innerhalb eines durch zwei Siebgrößen definierten Größenbereichs wird gegen die Partikelgröße aufgetragen, so dass die Partikelgrößenverteilung erhalten wird. Typische Größenangaben sind D₁₀, D₅₀ und D₉₀, wobei die Ziffern den Gewichtsanteil des Siebgutes in Prozent bezeichnen, der kleiner als die entsprechende Wertangabe ist.

Der Begriff "Schüttdichte" (engl.: bulk density, auch als "Schüttgewicht" bezeichnet) von Granulat oder Pulver wird in Masse pro Volumeneinheit angegeben. Die Schüttdichte ist definiert als die Masse vieler Materialpartikel bezogen auf das von ihnen belegte Gesamtvolumen. Sie wird gemessen, indem ein Behälter mit bekannten Volumen aufgefüllt und gewogen wird. Die Schüttdichte von in Pulver- oder Granulatform vorliegenden Substanzen wird nach der Internationalen Norm ISO 697 (frühere Ausgabe: DIN 53912) bestimmt. Der Begriff "Rütteldichte" (engl.: tapped density) bezeichnet die Dichte, die nach mechanischer Verdichtung des Pulvers oder des Granulats beispielsweise mittels Vibration des Behälters erzeugt wird. Sie wird nach DIN/ISO 787 Teil 11 ermittelt.

Das "Porenvolumen" eines porösen Materials bezeichnet das von Hohlräumen belegte, freie Volumen innerhalb des Materials. Das Porenvolumen wird beispielsweise mittels eines Porosimeters gemessen, wobei eine nicht benetzende Flüssigkeit (wie beispielsweise Quecksilber) unter Einwirkung eines äußeren Druckes in die Poren eines porösen Materials gegen die entgegenwirkenden Oberflächenspannungskräfte gepresst wird. Die dazu benötigte Kraft ist umgekehrt proportional zur Porengröße und daher kann neben dem Porengesamtvolumen auch die Porengrößenverteilung der Probe ermittelt werden. Die Quecksilberporosimetrie erfasst nur Porengrößen oberhalb von 2 nm (Mesoporen und Makroporen).

### Ausführungsbeispiel

Nachfolgend wird die Erfindung anhand eines Ausführungsbeispiels und einer Patentzeichnung näher erläutert. In der Zeichnung zeigt im Einzelnen in schematischer Darstellung:
- **Figur 1**: eine Ausführungsform der erfindungsgemäßen faseroptischen Streueinrichtung mit zylinderförmigem Streuelement als Diffusor in einer Seitenansicht im Schnitt,
- **Figur 2**: eine vergrößerte Detailansicht A der Sintermasse der Streueinrichtung von Figur 1,
- **Figuren 3-5**: Verfahrensschritte für die Herstellung der erfindungsgemäßen faseroptischen Streueinrichtung,
- **Figur 6**: ein Foto eines zylindrischen Streumusters bei einer Probe gemäß der Erfindung, und
- **Figur 7**: ein Foto eines zylindrischen Streumusters bei einer weiteren Probe.

### Beispiel einer faseroptischen Streueinrichtung

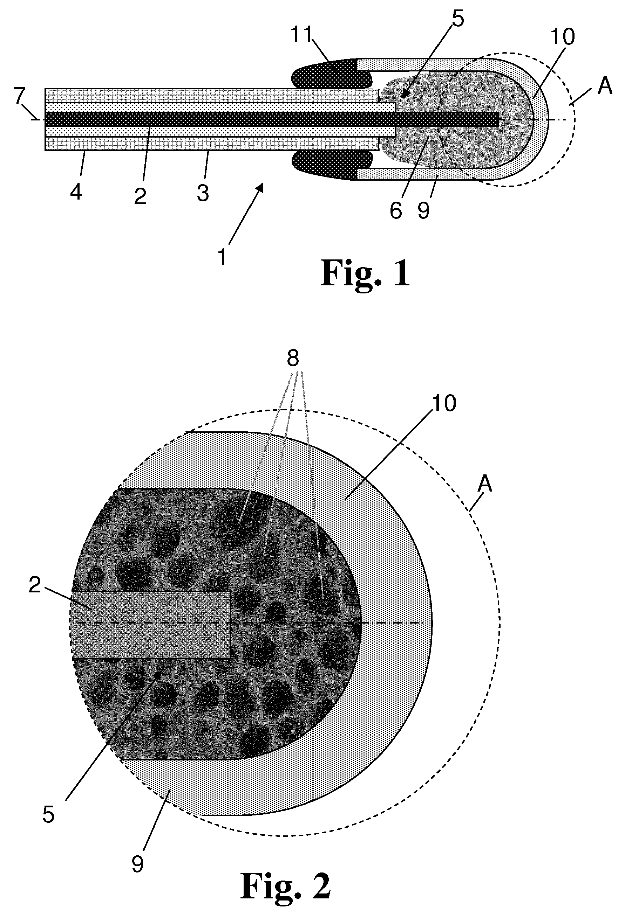

Die zylinderförmige, faseroptische Streueinrichtung von **Figur 1** umfasst eine optische Lichtleitfaser 1 mit einer Länge von etwa 2 m. Die Lichtleitfaser 1 hat einen Faserkern 2, einen den Faserkern umhüllenden Mantel 3 und eine Schutzumhüllung aus einem Kunststoffmantel 4. Der Faserkern 2 hat einen Durchmesser von 200 µm und besteht aus reinem, undotiertem Quarzglas. Der optische Mantel 3 besteht aus Quarzglas, das mit etwa 4 Gew.-% Fluor dotiert ist.

Am distalen Faserende 5 ist der Kunststoffmantel 4 vollständig und der optische Mantel 3 zum größten Teil entfernt. Die Länge des blanken Kerns 2 beträgt etwa 5 mm. Es ragt in die Bohrung einer verschmolzenen Glaskapsel 9 aus Quarzglas, von der das hintere Ende 10 zugschmolzen und das vordere Ende mit einem Klebepfropfen 11 verschlossen ist. Die Wandstärke der Glaskapsel beträgt etwa 500 µm. Die Mantelfläche des blanken Kerns 2 und die Kern-Stirnseite sind teilweise von einer Streumasse 6 bedeckt, die einen Hohlraum der Glaskapsel 9 nahezu vollständig füllt. Die Streumasse 6 besteht aus gesintertem, porenhaltigem undotiertem Quarzglas mit einer Porosität um 15%. Die maximale Schichtdicke des Streuelements 6 entspricht der Spaltweite zwischen der Innenwandung der Glashülle 9 und dem Kern 2; das sind im Ausführungsbeispiel etwa 500 µm.

In das dem distalen Faserende 5 gegenüberliegende Faserende (nicht dargestellt) wird Arbeitsstrahlung mit einer Wellenlänge vom 532 nm eingekoppelt, die in der Lichtleitfaser 1 transportiert und zum distalen Ende 5 geführt wird. Dort endet die Lichtführung durch Totalreflexion, da die Brechungsindizes der Gläser von Kern 2 und Streumasse 6 für die Arbeitsstrahlung etwa gleich groß sind (sie betragen jeweils etwa 1,46). Somit gelangt Arbeitsstrahlung in die Streumasse, die als Diffusor dafür wirkt und sie gleichmäßig verteilt. Die Streueinrichtung zeigt im Bereich der Streumasse 6 - bezogen auf die Mittelachse 7 - eine homogene zylinderförmige Lichtabstrahlung mit einem nach außen streuenden zylindrischen Muster.

Aus der Detailansicht von **Figur 2** sind die offenen Poren 8 der porösen Quarz-glas-Streumasse 6 erkennbar. Die Porengrößen liegen im Makroporenbereich mit Porengrößen im Bereich von 250 bis 10.000 nm. Die Poren haben ein kleines Aspektverhältnis (auch als "Strukturverhältnis" bezeichnet) das typischerweise weniger als 3 und vorzugsweise weniger als 2 beträgt. Unter dem "Aspektverhältnis" wird hier das Verhältnis von größter und kleinster Porenabmessung verstanden. Ein Aspektverhältnis von maximal 2 bedeutet demnach, dass die größte Porenabmessung nicht mehr als 2-mal größer ist als seine kleinste Porenabmessung. Die Poren 8 bewirken über die Länge des freigelegten Kerns 2 eine intensive homogene seitliche Ausleitung der im Kern 2 geführten Arbeitsstrahlung.

Der poröse Überzug des Kerns 2 mit der Streumasse 6 wird vorzugsweise anhand eines Verfahrens verwirklicht, das nachfolgend beispielhaft erläutert wird.

### Beispiel zur Herstellung splittriger SiO₂-Körnung (Probe 4)

Durch Vermahlen von Quarzglas werden SiO₂-Körnungsteilchen mit gebrochener und im Wesentlichen splittriger Morphologie erhalten. Durch Sieben der Körnung wird eine SiO₂-Körnung mit enger Teilchengrößenverteilung erzeugt, die durch einen D₁₀- Wert von etwa 45 µm und durch einen D₉₀-Wert von etwa 63 µm gekennzeichnet ist (Probe 4 in Tabelle 1). SiO₂-Nanoteilchen mit Teilchengrößen um 40 nm ("pyrogene Kieselsäure") werden homogen eingemischt.

### Beispiel zur Herstellung splittriger dotierter SiO₂-Körnung (Probe 8)

Durch Vermahlen von Quarzglas werden SiO₂-Körnungsteilchen mit gebrochener und im Wesentlichen splittriger Morphologie erhalten. Durch Sieben der Körnung wird eine SiO₂-Körnung mit enger Teilchengrößenverteilung erzeugt, die durch einen D₁₀- Wert von etwa 125 µm und durch einen D₉₀-Wert von etwa 250 µm gekennzeichnet ist. In die SiO₂-Körnung werden feinteilige Borsäure (H₄O₃) und SiO₂-Nanoteilchen mit Teilchengrößen um 40 nm ("pyrogene Kieselsäure") durch Trockenmischen homogen eingemischt. Der Anteil an Borsäure ist dabei so bemessen, dass sich beim Sintern unter oxidierenden Bedingungen B₂O₃ in einem Anteil von 1 Gew.-% (bezogen auf den gesamten Feststoffgehalt der Körnung) einstellt.

### Beispiel zur Herstellung von SiO₂-Körnung in Form von Granulat (Probe 10)

Bei der Synthese von synthetischem Quarzglas als Filtermaterial anfallende SiO₂-Sootpartikel mit einer Partikelgröße von weniger als 100 nm und einer spezifischen Oberfläche (nach BET) von etwa 30 m²/g (Schüttdichte von etwa 0,1 kg/l) werden für die Herstellung von SiO₂-Granulat eingesetzt.

Es wird ein SiO₂-Schlicker hergestellt, indem das hochreine, pyrogene erzeugte SiO₂-Sootmaterial in deionisiertem Wasser dispergiert wird. Die Dispersion wird dabei auf ein Litergewicht von 1380 g/l eingestellt. Die Schlickerviskosität beträgt 450 mPas. Unter Einsatz eines handelsüblichen Sprühtrockners wird der Schlicker bei einer Heißlufttemperatur von 400 °C und einem Schlickerdruck von 10,5 bar versprüht.

Dabei wird ein Sprühgranulat mit einem mittleren Korndurchmesser im Bereich vom 100 bis 300 µm erhalten. Der Feinanteil mit einem Durchmesser unterhalb von 100 µm wird herstellungsbedingt bereits bei der Sprühgranulation mittels eines Zyklons abgetrennt. Jedes Granulatteilchen liegt als Agglomerat einzelner sphärischer SiO₂-Primärteilchen vor, das durch leichten mechanischen Druck zerrieben und zerkleinert werden kann. Zwischen den SiO₂-Primärteilchen sind offene Hohlräume ausgebildet. Die spezifische Oberfläche (nach BET) des Sprühgranulats beträgt 30 m²/g und die Schüttdichte liegt bei 0,8 kg/l.

Zur Reduzierung des Sinterschwundes wird das Sprühgranulat vor seinem Einsatz thermisch auf eine Schüttdichte von 1,0 kg/l und eine spezifische Oberfläche (BET) im Bereich zwischen 13-14 m²/g verdichtet. Dabei bleibt die Granulat-Körnung opak.

Für Sprühgranulat ist ein mehr oder weniger abgeschlossener Hohlraum charakteristisch, der auch durch thermisches Verdichten nicht ohne weiteres zu beseitigen ist. Daher kann das Verdichten des Sprühgranulats bis zur vollständigen Verglasung der Granulat-Teilchengeführt werden, wobei der verbleibende Hohlraum im individuellen Granulatteilchen als Streuzentrum fungiert.

### Beispiele zum Herstellen der Streueinrichtung_

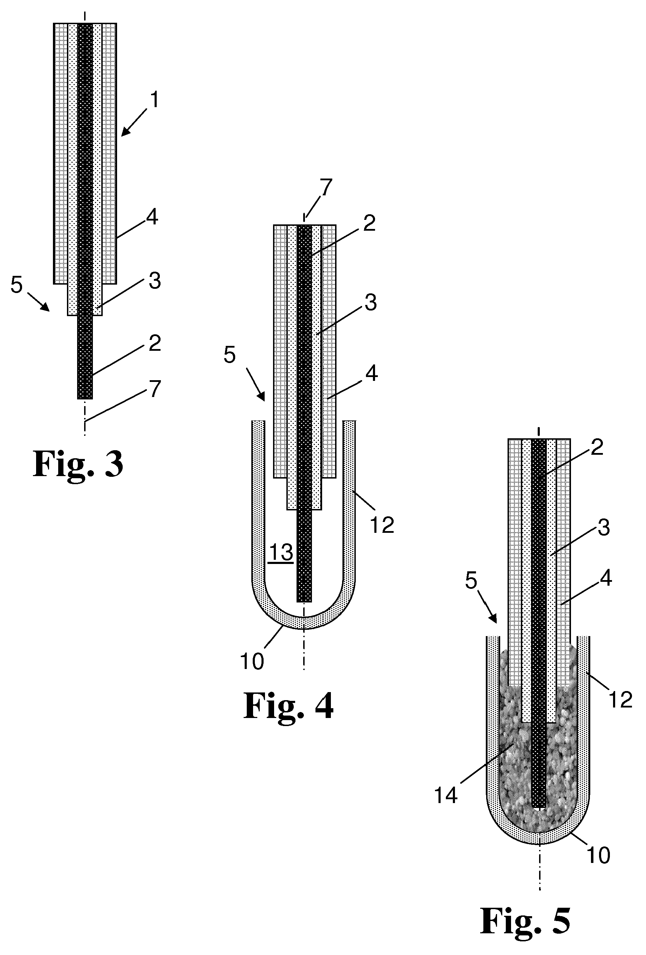

Die stirnseitig abgemantelte Lichtleitfaser 1 ist in **Figur 3** gezeigt. Sie wird mit dem abgemantelten Ende 5 nach unten so in eine einseitig geschlossene Kapillare 12 eingeführt, dass zwischen dem geschlossenen Ende 10 der Kapillare 12 und der Spitze des Kerns 2 ein Spalt von etwa 600 µm verbleibt. Der Ringspalt 13 zwischen der Zylindermantelfläche des Kerns 2 und der Innwandung der Kapillare 12 hat eine Spaltweite von etwa 500 µm. Dies zeigt schematisch **Figur 4****.**

In den Ringspalt 13 werden mittels eines Trichters oder mittels einer Pipette die SiO₂-Körnungsteilchen eingefüllt. Die so erhaltene Körnungs-Schüttung ist in **Figur 5** mit der Bezugsziffer 14 bezeichnet. Nachdem die Schüttung 14 mittels eines Rüttlers verdichtet worden ist, wird sie unter Einsatz einer Mikroplasma-Einrichtung gesintert, wie sie auch beim Spleißen optischer Fasern eingesetzt wird. Dabei erzeugt ein Plasmastrahl kurzzeitig (0,5 bis 2s) Temperaturen um 1400 ° C. Durch rasterweises Verschieben des Plasmastrahls wird die gesamte Quarzglas-Körnung 14 gesintert. Dabei wird die Wandung der Kapillare 12 derart erweicht, dass sie auf die sinternde Körnungs-Schüttung 14 aufschmilzt und nach außen offene Poren der Körnung verschließt. Die Kapillare 12 bildet danach die in den Figuren 1 und 2 schematisch gezeigte Glaskapsel 9.

Die nach Abschluss des Sintervorgangs erhaltene Streumasse 6 (Figuren 1 und 2) besteht aus Quarzglas und Poren. Sie umhüllt den blanken Kern 2 im Idealfall über die gesamte abgemantelte Länge von 5 mm. Die örtliche Verteilung der Poren 8 innerhalb der Streumasse 6 ist weitgehend homogen und die Porosität liegt bei 15% (Tabelle 1, Probe 4).

Abschließend wird das obere, noch offene Ende der Glaskapsel 9 (der ehemaligen Kapillare 12) durch den Klebstoffpfropfen 11 (Figur 1) verschlossen.

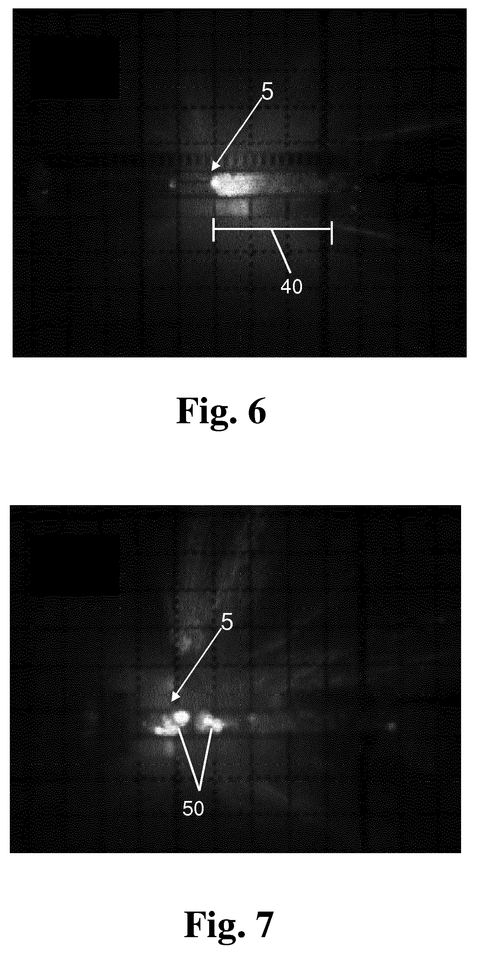

Die Strahlungsverteilung der sphärischen faseroptischen Streueinrichtung kann beispielsweise mittels eines optischen Detektors erfasst werden, der um das distale Ende der Lichtleitfaser 1 rotierbar ist. In einer einfacheren Messung wurde in das proximale Faserende rotes Laserlicht eingekoppelt und das Streumuster am distalen Ende erfasst. Die **Figuren 6 und 7** zeigen typische Ergebnisse dieser Streulichtmessungen anhand fotographischer Abbildungen der streuenden Faserenden vor dem Hintergrund von Millimeterpapier. Die dabei visuell erkennbare Beleuchtung des Faserendes ergibt sich als Kombination diskreter Reflexion und diffuser Streuung. Die Länge mit einer visuell als homogen identifizierbaren Beleuchtung wird erfasst und hier als Tiefe der Streuwirkung "T" ausgewertet (Tabelle 1).

Zur Optimierung der Streuwirkung und der mechanischen Eigenschaften der faseroptischen Streueinrichtung wurden Streumassen durch Sintern von Schüttungen unterschiedlicher Teilchengrößen aber ähnlicher Zusammensetzung erzeugt. Die Teilchengrößenverteilungen der jeweiligen Schüttungen und die damit erzielten Ergebnisse sind in **Tabelle** 1 angegeben:

**Tabelle 1 - Herstellung zylinderförmiger Streuelemente**

| **Nr.** | **Körnungs art** | **d [µm]** | **Soot [Gew-%]** | **Dotierung [Gew-%]** | **Füll-grad [%]** | **Porosität [%]** | **T [mm]** | **Ergeb gebnis** |
|---|---|---|---|---|---|---|---|---|
| **1** | - | - | 100 | 0 | 30 | >30 | <1 | - |
| **2** | S | 70 | 15 | 0 | 50 | >30 | <1 | - |
| **3** | S | 80 | 5 | 0 | 68 | 14 | 1 | 0 |
| **4** | B | 45-63 | 5 | 0 | 71 | 15 | 2 | ++ |
| **5** | B | 63-90 | 2,5 | 0 | 69 | 16 | 1,5 | 0 |
| **6** | B | 125-250 | 2,5 | 0 | 67 | 18 | 2 | + |
| **7** | B | 125-250 | 0 | 0 | 65 | 18 | 2 | 0 |
| **8** | B | 125-250 | 2,5 | 1 B₂O₃ | 67 | 16 | 3 | + |
| **9** | B | 250-315 | 2,5 | 0 | 60 | 25 | <1 | - |
| **10** | G | 100-250 | 0 | 0 | 60 | 25 | 3,2 | ++ |

Dabei bedeuten:
- Körnungsart:: S = handelsübliche, sphärische SiO₂-Körnung, B = gebrochene SiO₂-Körnung, G = Granulat (jeweils ohne Berücksichtigung von SiO₂-Soot)
- d:: Teilchengrößenverteilung in µm; Einzelwert: D90-Wert; Wertepaar: Bereich zwischen D10-Wert und D90-Wert (SiO₂-Körnung ohne Soot).
- Soot:: Gewichtsanteil der SiO₂-Nanoteilchen (SiO₂-Soot) an der SiO₂-Körnungsschüttung (Teilchengröße < 100 nm).
- Füllgrad:: Anteil des SiO₂-Feststoffes in der von der Schüttung innerhalb der Kapillare eingenommenen Volumens.
- T:: Tiefe (in mm) der Streuwirkung der gesinterten Streumasse in Faser-Längsrichtung gesehen.

In der Spalte "Ergebnis" sind die Homogenität der Streumasse, die Rissbehaftung und die Streuwirkung berücksichtigt. Dabei setzt sich die Symbolik der qualitativen Bewertung wie folgt um:
- "++": Homogenität, Rissbehaftung und Streuwirkung sind akzeptabel
- "+": zwei der drei Parameter sind akzeptabel
- "0": einer der drei Parameter ist akzeptabel,
- "-": keiner der drei Parameter ist akzeptabel.

Die Proben 1 und 2 zeigten beim Sintern der jeweiligen Schüttungen eine vergleichsweise hohe Schwindung, die zu einer überaus porösen Streumasse führte. Probe 1 zeigt darüber hinaus eine geringe Rieselfähigkeit, was das Auffüllen des Ringspalts 13 erschwerte. Auch bei Probe 9 erschwerten die vergleichsweise großen SiO₂-Teilchengrößen eine homogene Auffüllung des Ringspalts 13. Es ist aber davon auszugehen, dass dieses Problem durch eine größere Ringspaltweite entschärft werden kann.

Die Proben 3 bis 8 und 10 ergaben brauchbare Resultate bei mindestens einer der drei Qualitätskriterien, wobei bei keiner dieser Proben Risse, Abplatzungen oder Delaminationen zwischen Kern 2 und Streumasse beobachtet worden sind. Auch nach mehrmaligem Erhitzen der Proben auf eine Temperatur von 600 °C und anschließendem Abschrecken wurde keine mechanische Verschlechterung festgestellt. Die hohe mechanische Stabilität und thermische Belastbarkeit der faseroptischen Streueinrichtung wird darauf zurückgeführt, dass es zwischen dem Kern 2 der Lichtleitfaser 1 und der Streumasse keinen nennenswerten Unterschied im thermischen Ausdehnungskoeffizienten gibt. Dies kann wiederum bei den Proben 3 bis 7 und 10 auf den geringen Unterschied im thermische Ausdehnungskoeffizienten zurückzuführen sein, da der Kern aus undotiertem Quarzglas besteht und die Streumasse 6 eine Matrix aus undotiertem Quarzglas aufweist, und die Streuzentren im Wesentlichen als Partikeloberflächen und als Poren der Matrix vorliegen, die sich auf den Ausdehnungskoeffizienten der Streumasse 6 nicht oder nicht nennenswert auswirken.

Bei Probe 8 besteht zwischen Kern und Streumasse zwar ein Unterschied im thermischen Ausdehnungskoeffizienten. Dieser Nachteil wird aber durch die leichtere Sinterbarkeit der Streumasse aus dotiertem Quarzglas kompensiert, was sich in einer vergleichsweise großen Länge des mit Streumasse beschichteten, blanken Kerns zeigt.

Bei der Auswertung der axialen Streutiefe T ergaben sich Streulichtlängen im Bereich von weniger als 1 mm bis etwa 3,2 mm. Figur 6 zeigt die entsprechende Lichtverteilung für das aus SiO₂-Granulat erzeugte Sinterelement von Probe 10. Vom distalen Faserende 5 ausgehend nimmt die Intensität des Streulichts rasch ab, jedoch ohne erkennbare Inhomogenitäten infolge starker Reflexion. Die Tiefe der visuell erkennbaren Streuwirkung beträgt etwa 3,2 mm und ist mit dem Abmessungsbalken 40 gekennzeichnet. Demgegenüber sind bei der axialen Lichtverteilung von Figur 7 starke Reflexionen 50 zu erkennen, die auf große Körnungsteilchen der Probe 9 zurückgeführt werden können. Diese überstrahlen sogar den Beleuchtungseffekt, der sich ansonsten infolge des Austritts des Laserstrahls aus dem distalen Faserende 5 zeigt. Derartig starke Reflexionen können jedoch nicht nur durch feinere Körnung vermieden werden, sondern auch durch eine größere Schichtdicke des Sinterelements. Als Faustformel kann angegeben werden, dass die Schichtdicke mindestens fünfmal, besser zehnmal so groß sein sollte, wie die mittlere Korngröße.

## Patentansprüche

1. Faseroptische Streueinrichtung, mit einer Lichtleitfaser (1) aus Quarzglas zur Übertragung von Licht zu einem an einem distalen Endbereich (5) der Lichtleitfaser (1) vorgesehenen Diffusor, der Streuzentren enthält, **dadurch gekennzeichnet, dass** mindestens ein Teil der Streuzentren als offene Poren (8) in einer zu mindestens 90 Gew.-% aus SiO₂ bestehenden Sintermasse (6) ausgebildet ist, die mindestens teilweise von einer Glashülle (9) umgeben ist.

2. Faseroptische Streueinrichtung nach Anspruch 1, **dadurch gekennzeichnet, dass** die Sintermasse (6) zu mindestens 95 Gew.-% aus SiO₂ besteht.

3. Faseroptische Streueinrichtung nach Anspruch 1 oder 2, **dadurch gekennzeichnet, dass** die Glashülle (9) aus Quarzglas besteht und mit der Sintermasse (6) integral verbunden ist.

4. Faseroptische Streueinrichtung nach einem der vorhergehenden Ansprüche, **dadurch gekennzeichnet, dass** die Sintermasse (6) in Kontakt mit einem Faserkern (2) ist, und dass sich die Zusammensetzungen von Sintermasse (6) und Faserkern (2) in ihrem SiO₂-Gehalt um 1 Prozentpunkt oder weniger, vorzugsweise um weniger als 0,5 Prozentpunkte, unterscheiden.

5. Faseroptische Streueinrichtung nach einem der Ansprüche 1 bis 3, **dadurch gekennzeichnet, dass** die Sintermasse (6) eine Porosität von mindestens 10 %, vorzugsweise mindestens 15% aufweist.

6. Faseroptische Streueinrichtung nach einem der vorhergehenden Ansprüche, **dadurch gekennzeichnet, dass** das distale Ende (5) der Lichtleitfaser kegelförmig ausgebildet ist.

7. Verfahren zum Herstellen einer faseroptischen Streueinrichtung, bei der Licht in einer Lichtleitfaser (1) aus Quarzglas geführt und über einen lichtstreuenden Diffusor abgestrahlt wird, wobei zur Erzeugung des Diffusors auf einem Faserende (5) der Lichtleitfaser (1) eine Streumasse aufgebracht und diese zu dem Diffusor verfestigt wird, **dadurch gekennzeichnet, dass** das Aufbringen der Streumasse folgende Verfahrensschritte umfasst:
(a) Bereitstellen einer SiO₂-Körnung, die amorphe SiO₂-Teilchen enthält und die zu mindestens 90 Gew.-% aus SiO₂ besteht,
(b) Bereitstellen eines Hohlkörpers (12) aus Glas mit einer Hohlraumwandung, die einen nach außen offenen Hohlraum (13) umgibt,
(c) Bilden einer Schüttung (14) der SiO₂-Körnung in dem Hohlraum (13) und Einbringen des Faserendes (5) in den Hohlraum (13), so dass mindestens ein Teil des Faserendes (5) in die Schüttung (14) hineinragt.
(d) thermisches Verdichten der Schüttung (14) unter Ausbildung einer porenhaltigen und zu mindestens 90 Gew.-% aus SiO₂ bestehenden Sintermasse (6), die mindestens teilweise von einer Glashülle (9) umgeben ist.

8. Verfahren nach Anspruch 7, **dadurch gekennzeichnet, dass** die SiO₂-Körnung und die Sintermasse (6) zu mindestens 95 Gew.-% aus SiO₂ bestehen.

9. Verfahren nach einem der Ansprüche 7 oder 8, **dadurch gekennzeichnet, dass** die amorphe SiO₂-Körnung SiO₂-Teilchen mit Teilchengrößen im Bereich bis maximal 500 µm, vorzugsweise maximal 350 µm, enthält, wobei SiO₂-Teilchen mit Teilchengrößen im Bereich zwischen 100 µm und 200 µm den größten Volumenanteil ausmachen.

10. Verfahren nach Anspruch 9, **dadurch gekennzeichnet, dass** die amorphe SiO₂-Körnung SiO₂-Nanoteilchen in einem Gewichtsanteil - bezogen auf ihren gesamten Feststoffgehalt - im Bereich von 1 bis 10 % enthält.

11. Verfahren nach einem der Ansprüche 7 bis 10, **dadurch gekennzeichnet, dass** eine Schüttung (14) mit einem Füllgrad von weniger als 85 %, bevorzugt im Bereich von 60 bis 80 %, erzeugt wird.

12. Verfahren nach einem der Ansprüche 7 bis 11, **dadurch gekennzeichnet, dass** eine Schüttung (14) erzeugt wird, die zwischen dem Faserende (5) und der Hohlraumwandung eine Schüttungsschicht mit einer Schichtdicke im Bereich von 0,1 bis 3 mm aufweist.

13. Verfahren nach Anspruch 12, **dadurch gekennzeichnet, dass** innerhalb der Schüttungsschicht ein axialer Gradient in der Dichte der Schüttung oder in der Zusammensetzung der SiO₂-Körnung eingestellt wird, derart, dass sich nach dem thermischen Verdichten der Schüttung ein Dichtegradient zwischen einer geringeren Dichte in einem faserendnahen Bereich und einer höheren Dichte in einem faserendfernen Bereich des Hohlkörpers einstellt.

14. Verfahren nach einem der Ansprüche 7 bis 13, **dadurch gekennzeichnet, dass** die SiO₂-Körnung eine erste Körnungsart mit einem ersten Brechungsindex und einer ersten Viskosität sowie zweite Körnungsart mit einem zweiten Brechungsindex und einer zweiten Viskosität aufweist, wobei sich der erste Brechungsindex und der zweite Brechungsindex voneinander unterscheiden und wobei die zweite Viskosität niedriger ist als die erste Viskosität.

15. Verfahren nach einem der Ansprüche 7 bis 14, **dadurch gekennzeichnet, dass** ein als Rohr oder Hohlkugel ausgebildeter Hohlkörper (12) eingesetzt wird.
